# EUROPÄISCHE PATENTSCHRIFT

(11) **EP 2 908 785 B1**
(45) Veröffentlichungstag und Bekanntmachung des Hinweises auf die Patenterteilung: **17.05.2017**
(21) Anmeldenummer: 13795177.8
(22) Anmeldetag: 09.10.2013
(51) Int. Cl.: A61F 5/01, A61F 5/30

(54) **ORTHESE**
ORTHOSIS
ORTHÈSE

(30) Priorität: 16.10.2012 DE 102012218804
(43) Veröffentlichungstag der Anmeldung: 26.08.2015
(73) Patentinhaber: Ortema GmbH, 71706 Markgröningen (DE)
(72) Erfinder: SEMSCH, Hartmut, 70374 Stuttgart (DE); BRÜGGEMANN, Gert-Peter, 50858 Köln (DE); KÜSEL-FELDKER, Martin, 50933 Köln (DE)
(74) Vertreter: Kohler Schmid Möbus Patentanwälte
(86) Internationale Anmeldenummer: PCT/EP2013/071005
(87) Internationale Veröffentlichungsnummer: WO 2014/060251

(56) Entgegenhaltungen:
- WO-A1-2011/154779
- GB-A- 2 327 044
- US-A1- 2012 143 111

## Beschreibung

Die vorliegende Erfindung betrifft eine Orthese zum Stabilisieren, Führen und/oder Korrigieren eines Gelenks.

Orthesen werden seit langem als medizinische Hilfsmittel für eine funktionelle Stabilisierung, Entlastung Führung und/oder Korrektur von Gelenken eingesetzt. So werden Knieorthesen beispielsweise nach einer Versorgung von Rupturen des vorderen oder hinteren Kreuzbandes, nach Seitenbandläsionen sowie bei Meniskus- und Knorpelschäden eingesetzt.

Die Orthesen weisen üblicher Weise ein Gelenkschienenpaar mit jeweils einem ersten und einem zweiten Schienenteil auf, die an ihren einander zugewandten Enden über ein Orthesengelenk gelenkig miteinander verbunden sind. Die beiden Schienenteile sind um eine Gelenkachse relativ zueinander verschwenkbar und über Befestigungsmittel, beispielsweise Gurte oder Klettbänder, an einem jeweiligen Gliedmaßenabschnitt, etwa dem Ober- und Unterschenkel, befestigbar.

Obwohl die Orthesen entweder in verschiedenen Größen verfügbar sind oder auch individuell angepasst werden, kann das mit der Orthese zu versorgende Gelenk häufig nicht in dem gewünschten Maße stabilisiert bzw. entlastet oder aktiv korrigiert werden. Dies kann insbesondere bei postoperativen oder auch posttraumatischen Schwellungen im Gelenkbereich sowie bei Valgus- oder Varusfehlstellungen des zu versorgenden Gelenks der Fall sein. Bei der Valgusfehlstellung weicht der distale Gliedmaßenabschnitt, d.h. beispielsweise der Unterschenkel, über das Normalmaß hinaus von der Mittelachse der Gliedmaße nach außen weg, während unter der Varusfehlstellung eine Abweichung nach innen verstanden wird. Die unzureichende Stabilisierung, Führung, Entlastung oder auch aktive Korrektur des zu versorgenden Gelenks bzw. des gelenknahen Bandapparates kann zu einem protrahierten Heilverlauf und ggf. zu einer dauerhaften Gelenkinstabilität führen.

Aus der WO 2011/154779 A1 ist eine verbesserte Orthese zum Stabilisieren, Führen und/oder Entlasten eines Gelenks bekannt geworden, die eine Gelenkschiene mit einem ersten und einem zweiten Schienenteil umfasst, welche um eine Gelenkachse relativ zueinander verschwenkbar sind. Die Orthese weist eine Korrekturpelotte auf, die durch ein relatives Verschwenken der beiden Schienenteile entlang der Gelenkachse in Richtung auf und gegen das mit der Orthese zu stabilisierende Gelenk verstellbar ist.

Ausgehend von dem vorgenannten Stand der Technik ist es Aufgabe der Erfindung, eine Orthese bereitzustellen, die eine verbesserte seitliche Gelenkstabilisierung, -führung,-entlastung und/oder aktive Korrektur ermöglicht.

Die die Orthese betreffende Aufgabe wird durch eine Orthese mit den in Patentanspruch 1 angegebenen Merkmalen gelöst. Vorteilhafte Weiterbildungen der Erfindung sind Gegenstand von Unteransprüchen.

Bei der erfindungsgemäßen Orthese kann durch die entlang der Gelenkachse translatorisch bewegbar angeordnete Korrekturpelotte (=Druckglied) ein definiertes Valgus- oder Varusmoment in Abhängigkeit von einer jeweiligen Schwenkstellung der beiden Schienenteile zueinander, d.h., in Abhängigkeit von einer jeweiligen Flexion des mit der Orthese versorgten Gelenks, auf das Gelenk ausgeübt werden. So kann die Korrekturpelotte beispielsweise in Neutralstellung (0° des Gelenks nach der sogenannten Neutral-Null-Methode, dem standardisierten orthopädischen Bewertungsindex für Gelenke) oder auch in Beugestellung (Flexion) des Gelenks, beispielsweise bei einem Kniewinkelbereich von 0-25° oder ungefähr 30°, in Richtung auf und gegen das zu versorgende Gelenk geführt werden. Im Falle eines Kniegelenks kann die Korrekturpelotte dabei beispielsweise gegen den condylus medialis femoris, also den zur Körpermitte hin gerichteten distalen Gelenkfortsatz des Oberschenkelknochens, geführt bzw. gepresst werden (Valgusmoment - Korrektur im Sinne von Varus). Dadurch kann das mit der Orthese zu versorgende Gelenk seitlich insgesamt wirkungsvoller stabilisiert, entlastet, geführt und/oder aktiv korrigiert werden. Darüber hinaus können dadurch Sekundärschäden am Gelenk oder dem das Gelenk umgebenden Bandapparat wirkungsvoll vermieden werden. Der individuelle Heilungsverlauf und Heilungserfolg kann dadurch auf einfache und wirtschaftliche Weise verbessert werden. Die Korrekturpelotte kann insbesondere Teil eines Korrekturgelenks der Orthese sein, über das die beiden Schienenteile der Gelenkschiene gelenkig miteinander verbunden sind.

Die axiale Bewegung der Korrekturpelotte entlang der Gelenkachse wird erfindungsgemäß dadurch aus der Verschwenkbewegung der beiden Schienenteile abgeleitet, dass eines der beiden Schienenteile eine drehfest mit diesem Schienenteil verbundene Nockenkurve und das jeweils andere der beiden Schienenteile einen Läufer (=Steuerglied) aufweist, das an der Nockenkurve anliegt. Der Läufer ist dabei an dem jeweils anderen Schienenteil vorzugsweise in axialer Richtung starr befestigt/gelagert. Die Nockenkurve oder der Läufer ist an der Korrekturpelotte axial lagefixiert, so dass die Korrekturpelotte bei einem Verschwenken der beiden Schienenteile, und der daraus resultierenden Relativbewegung der Nockenkurve und des an der Nockenkurve anliegenden Läufers, entlang der Gelenkachse translatorisch bewegt wird. D.h., die Korrekturpelotte führt eine Vorwärtshubbewegung aus, die entlang der Gelenkachse der Orthese in Richtung auf und gegen das axial fehlgestellte (=Valgus- oder Varusfehlstellungen) und orthetisch versorgte Gelenk gerichtet ist. Das Verhältnis zwischen einer jeweiligen Änderung eines von den beiden Schienenteilen eingeschlossenen Winkels und einer daraus abgeleiteten axialen Verstellbewegung der Korrekturpelotte kann auf einfache Weise anhand einer Flankensteigung des Nockens gegenüber der Gelenkachse eingestellt (vorgegeben) werden. Anhand der Nockenhöhe kann darüber hinaus ein (Gesamt-)Hub der Korrekturpelotte in axialer Richtung vorgegeben werden. Unter der Nockenhöhe wird der Abstand der Nockenspitze des Nockens von der (axialen) Nennhöhe der Nockenkurve verstanden, die mit der Ruhestellung der Korrekturpelotte korrespondiert.

Die Rückwärtsbewegung der Korrekturpelotte von deren (aktiver) Vorhubendlage in deren axiale Ruhelage kann nach der Erfindung rein passiv erfolgen. Es versteht sich, dass die Korrekturpelotte alternativ auch mittels eine Rückstellfeder oder dergl. aus der Vorhubendlage in die Ruhelage zurückbewegbar sein kann.

Nach der Erfindung kann die Nockenkurve insbesondere von einem Nockenring gebildet sein. Der Nockenring ist an der Korrekturpelotte vorzugsweise lösbar befestigt. Dies bietet einerseits fertigungstechnische Vorteile. Andererseits kann der Nockenring im Falle eines Verschleißes oder auch bei geänderten medizinischen Erfordernissen auf einfache Weise ausgetauscht bzw. im Bedarfsfalle auch ersatzlos entfernt werden. Es versteht sich, dass die Nockenkurve auch von einer Nockenscheibe ausgebildet sein kann.

Der Nockenring kann an der Korrekturpelotte in seiner axialen Position und in seiner Drehlage auf der Korrekturpelotte insbesondere variabel stufenlos einstellbar sein. Durch die Wahl der Nockenprofilhöhe kann die Vorhubendlage der Korrekturpelotte auf das individuell erforderliche Maß und in Abhängigkeit von einer jeweiligen relativen Verschwenkstellung der beiden Schienenteile zueinander bedarfsgerecht (fein-)eingestellt und justiert werden. Durch eine Verdrehung des Nockenrings können darüber hinaus die Steuerzeiten eingestellt werden. Dies kann insbesondere bei einem geschwollenen Knie- oder Ellenbogengelenk von Vorteil sein. So kann die Korrekturpelotte beispielsweise (nur) zwischen einer Beugestellung von 20 Grad bis zur Neutralstellung des zu stabilisierenden Gelenks gegen das Kniegelenk oder das Ellenbogengelenk gedrückt werden. Darüber hinaus lässt sich ein gewünschter Anpressdruck der Korrekturpelotte gegen das zu stabilisierende Gelenk (=Valgus- oder Varusmoment) dadurch auf einfache Weise einstellen. Vor allem kann bei der Beugung, z.B. bei einem Kniewinkel von größer 80°, die Korrektur des Orthesengelenks bedarfsweise entfallen, so dass es zu einer seitlichen Entlastung des Kniegelenks im Sitzen kommt.

Eine Überbeanspruchung des mit der Orthese zu stabilisierenden Gelenks kann erfindungsgemäß dadurch vermieden werden, dass die korrigierende Orthese einen nur begrenzten Bewegungsumfang im Gelenk ermöglicht. Im konstruktiv einfachsten Falle kann dies dadurch erreicht werden, das der Nockenring einen oder mehrere Anschläge für den Läufer aufweist.

Die Orthese weist unter konstruktiven Gesichtspunkten bevorzugt ein ringförmiges (Dreh-)Gelenk auf.

Die Korrekturpelotte kann insbesondere an einem vom (Dreh-)Gelenk umgriffenen Lagerteil axial verschiebbar gelagert sein. Dies ermöglicht eine zur Gelenkachse koaxiale Verstellung der Korrekturpelotte. Das Lagerteil ist dabei bevorzugt mit einem der beiden Schienenteile der Gelenkschiene drehfest verbunden.

Nach einer besonders bevorzugten Weiterbildung der Erfindung weist die Korrekturpelotte zumindest einen Lagerzapfen auf, der sich durch eine Bohrung des Lagerteils hindurcherstreckt und über den die Korrekturpelotte in der Bohrung in axialer Richtung (gleit-) gelagert ist. Die Bohrung oder der Lagerzapfen kann dabei mit einer PTFE- (Polytetrafluorethylen) Beschichtung bzw. einer PTFE-Gleithülse versehen sein. Die Korrekturpelotte kann nach der Erfindung auch zwei oder mehrere solcher Lagerzapfen aufweisen.

Der Lagerzapfen kann insbesondere einenends mit einem Halteteil versehen sein, auf dem der Nockenring lösbar befestigt ist. Das Halteteil kann insbesondere mit einem Haltebund für den Nockenkörper versehen sein. Dadurch kann ein axiales Entfernen bzw. Herausfallen der Korrekturpelotte aus der Bohrung des Lagerteils auf einfache Weise unterbunden werden. Das Halteteil kann bei dieser Ausführungsform somit einen Anschlag für eine axiale Vorwärtshubbewegung der Korrekturpelotte ausbilden.

Der Läufer kann erfindungsgemäß eine kugelgelagerte Rolle aufweisen. Dadurch kann eine gegenüber Störungen besonders robuste Funktion der Orthese erreicht werden. Darüber hinaus können in der Praxis störende Verstellgeräusche zuverlässig vermieden werden.

Die Korrekturpelotte ist bevorzugt mit einer Polsterauflage versehen. Dies ist für den Tragekomfort und die Patientensicherheit von Vorteil. Darüber hinaus kann dadurch Druckstellen zuverlässig entgegengewirkt werden.

Der Tragekomfort wie auch die Patientensicherheit können dadurch nochmals weiter verbessert werden, dass die Polsterauflage an der Korrekturpelotte frei verdrehbar angeordnet ist. Dies kann nach der Erfindung beispielsweise über einen an der Korrekturpelotte verdrehbar gelagerten Drehteller oder dergl. erreicht werden.

Es versteht sich, dass die Schienenteile jeweils Befestigungsmittel für Gliedmaßenabschnitte, beispielsweise Spanngurte , aufweisen können. Die Befestigungsmittel sind dabei aus Komfortgründen bevorzugt mit Klettverschlüssen versehen.

Die Orthese kann vorteilhafter Weise aus einem Leichtmetall, insbesondere aus Aluminium oder einer Aluminiumlegierung, oder aus einem Carbonfaserwerkstoff oder aus unzerbrechlichem Kunststoff gefertigt sein. Die Schienenteile können dabei im Wesentlichen aus einem formgebogenem Drahtprofil gebildet sein.

Nach einer Weiterbildung der Erfindung weist die Orthese eine weitere Gelenkschiene auf. Die weitere Gelenkschiene kann insbesondere von der erste Gelenkschiene beabstandet angeordnet sein und ebenfalls zwei Schienenteile aufweisen. Dadurch kann das mit der Orthese zu stabilisierende Gelenk beidseits gestützt, stabilisiert, und/oder korrigiert werden. Die weitere Gelenkschiene kann erfindungsgemäß ebenfalls eine in der vorstehend erläuterten Weise ausgebildete Korrekturpelotte aufweisen. Die Korrekturpelotten der beiden Gelenkschienen können insbesondere synchron oder auch unabhängig voneinander gleich- oder gegensinnig axial verstellbar angeordnet sein.

Eine erfindungsgemäßes Korrekturgelenk für eine vorstehend erläuterte Orthese umfasst die Gelenkachse und die entlang der Gelenkachse axial verstellbare Korrekturpelotte. Das Korrekturgelenk weist vorzugsweise zwei Gelenkteile auf, die um die Gelenkachse gegeneinander verschwenkbar sind. An den beiden Gelenkteilen können die beiden Schienenteile der Orthese, vorzugsweise lösbar, befestigbar sein. Die entlang der Gelenkachse axial verstellbare Korrekturpelotte ist somit integraler Bestandteil des Korrekturgelenks und mittels eines relativen Verschwenkens der beiden Gelenkteile um die Gelenkachse entlang der Gelenkachse (zwangsweise) verschiebbar.

Nachfolgend wird die Erfindung anhand eines in der Zeichnung wiedergegebenen Ausführungsbeispiels näher erläutert.

In der Zeichnung zeigen:
- Fig. 1: eine Orthese mit einer Gelenkschiene, die zwei gegeneinander verschwenkbare Schienenteile aufweist, und mit einer Korrekturpelotte, das durch ein Verschwenken der Schienenteile entlang der Gelenkachse axial verstellbar ist, in einer ausschnittsweisen Schnittdarstellung;
- Fig. 2: die Orthese aus Fig. 1 mit in Vorhubendlage angeordneter Korrekturpelotte, in einer ausschnittsweisen Schnittdarstellung;
- Fig. 3: die Orthese aus Fig. 1, in einer ausschnittsweisen Seitenansicht; und
- Fig. 4: einen Nockenring der Orthese gemäß Fig. 1, über den das Druckglied mit den beiden Schienenteilen der Gelenkschiene bewegungsgekoppelt ist, in einer freigestellten perspektivischen Ansicht.

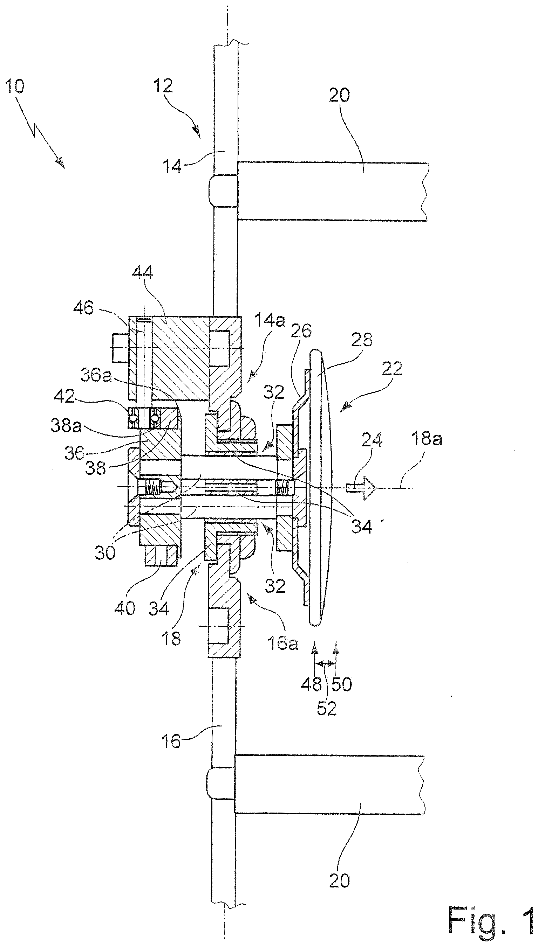

In **Fig. 1** ist eine erfindungsgemäße Orthese **10** in einem ausschnittsweisen Längsschnitt gezeigt. Die Orthese 10 dient dem Stabilisieren eines nicht näher wiedergegebenen Kniegelenks und weist eine Gelenkschiene **12** mit einem ersten und einem zweiten Schienenteil **14, 16** auf. Die beiden Schienenteile sind an ihren einander zugewandten Enden **14a, 16a** über ein als Drehgelenk ausgebildetes Korrekturgelenk **18** miteinander verbunden. Befestigungsmittel **20** dienen einer Befestigung der Orthese am Ober- bzw. Unterschenkel einer zu behandelnden Person (nicht gezeigt). Die Befestigungsmittel 20 sind in Ihrer jeweiligen axialen Lage an den Schienenteilen 14, 16 variabel einstellbar.

Das Korrekturgelenk 18 weist eine Gelenkachse **18a** auf, um die die beiden Schienenteile 12, 14 relativ zueinander verschwenkbar sind. An der Gelenkschiene 12 ist eine Korrekturpelotte **22** angeordnet, die durch eine Verschwenkbewegung der beiden Schienenteile 12, 14 im Drehgelenk 18 entlang der Gelenkachse 20 in Richtung auf und gegen das zu stabilisierende Kniegelenk bewegbar ist, wie dies mit dem Pfeil **24** verdeutlicht ist. Die Druckpelotte 22 weist einenends einen Drehteller **26** mit einer Polsterauflage **28** auf.

Die Korrekturpelotte 22 weist zwei voneinander beabstandet angeordnete Lagerzapfen **30** mit jeweils zylindrischem Querschnitt auf. Die Lagerzapfen 30 erstrecken sich durch Bohrungen **32** eines von dem Drehgelenk 18 umfangsseitig umgriffenen Innenlagerteils **34** hindurch. Die Bohrungen 32 des Innenlagerteils 34 sind jeweils mit Gleithülsen **34**' versehen, an denen die Lagerzapfen 30 der Korrekturpelotte 22 im Gleitspiel-Formschluss axial verschiebbar geführt sind. Die Gleithülsen 36 können insbesondere aus PTFE (Polytetrafluorethylen) oder einem PTFE-basierten Werkstoff bestehen.

An den Lagerzapfen 30 ist einenends ein scheibenförmiges Halteteil **36** angeordnet. Auf dem Halteteil 36 ist ein Nockenring **38** mittels mehrerer Schrauben **40** lösbar befestigt. Der Nockenring 38 ist auf dem Halteteil 36 drehfest und axial unverschiebbar gehalten angeordnet und kann durch Lösen der Schrauben 40 in seiner Drehlage relativ zur Gelenkachse 18a sowie in seiner axialen Position auf dem Halteteil 36 stufenlos variabel angeordnet werden.
Der Nockenring 38 liegt, wie aus Fig. 1 hervorgeht, vorliegend an einem Bund 36a des Halteteils 36 an.

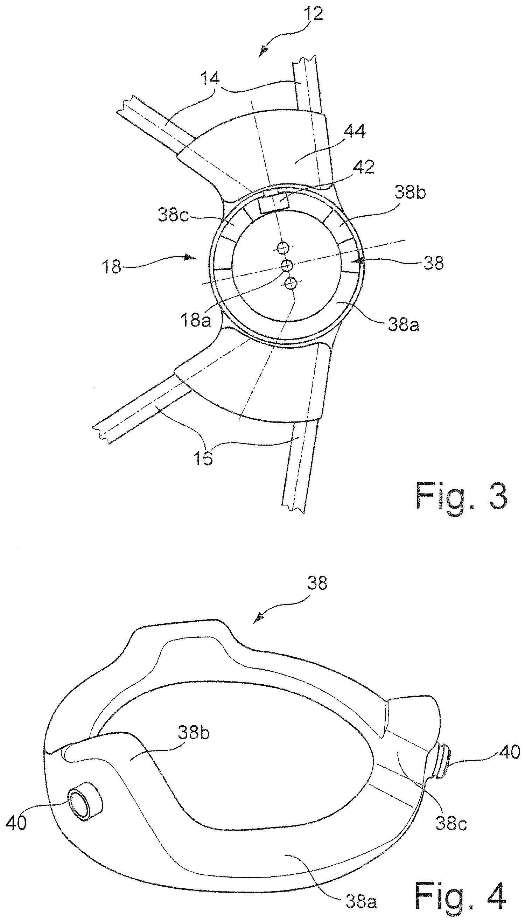

Der Nockenring 38 weist eine Nockenkurve 38a, d.h. eine Steuerkurve mit einem Nocken (nicht gezeigt), auf, die in den Figuren 3 und 4 näher wiedergegeben ist.

An der Nockenkurve 38a liegt ein Läufer **42** an. Der Läufer 42 ist an einem Lagerbock **44** des ersten Schienenteils 14 angeordnet und als eine um Rollenachse **46** frei verdrehbare kugelgelagerte Rolle ausgeführt.

Bei einem Verschwenken der beiden Schienenteile 14, 16 im Drehgelenk 18 rollt der Läufer 42 somit auf der unter dem Läufer 42 vorbeigeführten Nockenkurve 38a ab. Sobald der Läufer 42 gegen den Nocken (nicht gezeigt) geführt ist, wird der Nockenring 38 zusammen mit der Korrekturpelotte 22 aus der hier gezeigten Ruhestellung **48** entlang der Gelenkachse 18a des Drehgelenks 18 in Richtung 24 auf und gegen das zu stabilisierende Knie bewegt.
Eine Vorhubendlage der Korrekturpelotte 22 ist mit **50** bezeichnet. Die Korrekturpelotte 22 weist vorliegend einen Hub **52** von vorliegend ungefähr 10 mm auf. Der Hub 52 entspricht dem axialen Abstand zwischen der Neutralstellung 48 und der Vorhubendlage 50 der Korrekturpelotte 22.

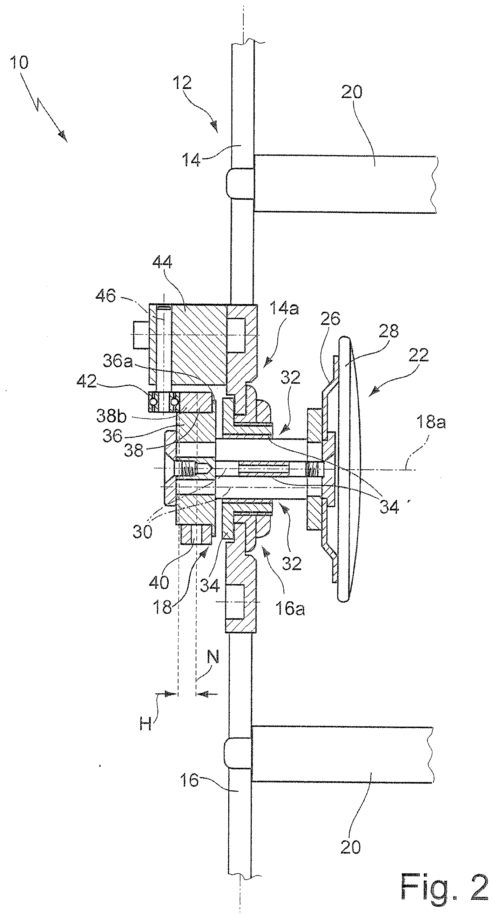

**Fig. 2** zeigt die Orthese 10 mit in eine Vorhubendlage 50 verstellter Korrekturpelotte 22 Die beiden Schienenteile 14, 16 sind in der hier gezeigten nicht gegeneinander abgewinkelt. Der Läufer 42 liegt an dem Nocken 38b der Nockenkurve 38a des Nockenrings 38 an. Die Nocke weist gegenüber der Nennhöhe N der Nockenkurve eine Nockenhöhe H auf, die dem Hub 52 der Korrekturpelotte 22 entspricht. Die Korrekturpelotte 22 liegt in der gezeigten Vorhubendlage 50 dem mit der Orthese zu stabilisierenden Kniegelenk vorzugsweise im Bereich des medialen bzw. lateralen Condylus an.

**Fig. 3** zeigt die Orthese 10 in einer ausschnittsweisen Seitenansicht bei entfernter Korrekturpelotte. Der Nockenring 38 mit dem daran stirnseitig aufliegenden Läufer 42 ist gut zu erkennen. Die Schienenteile 14, 16 sind im wesentlichen als profilierte Leichtmetallstreben ausgebildet. Die Schienenteile 14, 16 können auch aus einem anderen Werkstoff, insbesondere aus einem Carbonfaserwerkstoff, gebildet sein.

**Fig. 4** zeigt den Nockenring 38 in einer freigestellten perspektivischen Ansicht. Die Nockenkurve 38a umfasst den Nocken 38b, über den die Korrekturpelotte axial verstellbar ist. Ein Anschlag **38c** für den Läufer 42 dient einer Begrenzung des Bewegungsumfang der beiden Schienenteile 14, 16 im Drehgelenk 18 (Fig. 1).

Die vorstehend beschriebene Orthese eignet sich insbesondere bei allen medizinischen Indikationen, bei denen eine Führung, Stabilisierung, Entlastung und/oder Korrektur des Knie- oder Ellenbogengelenks bzw. eine Entlastung des Gelenks notwendig ist, wie z. B. bei einer höhergradigen und/oder komplexen Instabilität des Kniegelenks, bei Meniskusverletzungen, Knorpelschäden, sowie zur funktionellen prä- und/oder postoperativen Versorgung, insbesondere nach Rupturen des Bandapparates mit oder ohne Achs-Fehlstellungen. Die Orthese weist ein als Drehgelenk ausgebildetes Korrekturgelenk mit einem ersten und einem zweiten Schienenteil auf, die um eine Gelenkachse relativ zueinander verschwenkbar sind. Das Korrekturgelenk weist eine Korrekturpelotte auf, die mittels einer aus der Verschwenkbewegung der beiden Schienenteile abgeleiteten Verstellbewegung entlang der Gelenkachse in Richtung auf und gegen das zu stabilisierende/korrigierende Gelenk bewegbar ist.

## Patentansprüche

1. Orthese (10) zum Stabilisieren, Führen und/oder Entlasten eines Gelenks, umfassend
eine Gelenkschiene (12) mit einem ersten und einem zweiten Schienenteil (14, 16), die um eine Gelenkachse (18a) relativ zueinander verschwenkbar sind, und
eine Korrekturpelotte (22), die durch ein relatives Verschwenken der beiden Schienenteile (14, 16) entlang der Gelenkachse (18a) in Richtung auf und gegen das zu stabilisierende Gelenk verstellbar ist,
**dadurch gekennzeichnet, dass**
eines der beiden Schienenteile (14, 16) eine Nockenkurve (38a) aufweist, wobei das jeweils andere der beiden Schienenteile (14, 16) einen an der Nockenkurve geführten Läufer (42) aufweist und wobei die Nockenkurve (38a) oder der Läufer (42) an der Korrekturpelotte (22) axial lagegesichert ist.

2. Orthese nach Anspruch 1, **dadurch gekennzeichnet, dass** die Nockenkurve (38a) durch einen Nockenring (38) gebildet ist, der an der Korrekturpelotte lösbar befestigt ist.

3. Orthese nach Anspruch 2, **dadurch gekennzeichnet**, das der Nockenring (38) in seiner axialen Position und in seiner Drehlage relativ zur Korrekturpelotte (22) variabel auf der Korrekturpelotte (22) anordenbar ist.

4. Orthese nach einem der Ansprüche 2 oder 3, **dadurch gekennzeichnet, dass** der Nockenring (38) einen oder mehrere Anschläge (38c) für den Läufer (42) aufweist.

5. Orthese nach einem der vorhergehenden Ansprüche, **dadurch gekennzeichnet**, das die Gelenkschiene (12) ein auf der Gelenkachse (18a) angeordnetes Lagerteil (34) aufweist, an der die Korrekturpelotte (22) axial verschiebbar gelagert ist.

6. Orthese nach Anspruch 5, **dadurch gekennzeichnet, dass** die Korrekturpelotte (22) einen oder mehrere, vorzugsweise zylindrische, Lagerzapfen (30) aufweist, die in Bohrungen (32) des Lagerteils (34) gleitgelagert sind.

7. Orthese nach Anspruch 6, **dadurch gekennzeichnet, dass** die Lagerzapfen (30) einenends mit einem Halteteil (36) versehen sind, auf dem der Nockenring (38) lösbar befestigt ist.

8. Orthese nach einem der vorhergehenden Ansprüche, **dadurch gekennzeichnet, dass** der Läufer (42) eine kugelgelagerte Rolle aufweist.

9. Orthese nach einem der vorhergehenden Ansprüche, **dadurch gekennzeichnet, dass** die Korrekturpelotte (22) einen Drehteller (26) mit einer Polsterauflage (28) aufweist.

10. Orthese nach einem der vorhergehenden Ansprüche, **gekennzeichnet durch** eine weitere Gelenkschiene (12) mit einem ersten und einem zweiten Schienenteil (14, 16).

## Claims

1. Orthesis (10) for stabilising, guiding and/or relieving a joint, comprising
a joint rail (12) having a first and a second rail portion (14, 16) which can be pivoted relative to each other about a joint axle (18a) and
a correction pad (22) which can be adjusted by a relative pivoting of the two rail portions (14, 16) along the joint axle (18a) in the direction towards and counter to the joint to be stabilised, **characterized in that**
one of the two rail portions (14, 16) has a cam curve(38a), wherein the respective other of the two rail portions (14, 16) has a rotor (42) which is guided on the cam disc, and wherein the cam disc (38a) or the rotor (42) is axially fixed in position on the correction pad (22).

2. Orthesis according to claim 1, **characterised in that** the cam curve(38a) is formed by a cam ring (38) which is fixed to the correction pad in a releasable manner.

3. Orthesis according to claim 2, **characterised in that** the cam ring (38) can be arranged on the correction pad (22) so as to be variable in terms of the axial position thereof and in terms of the rotational position thereof relative to the correction pad (22).

4. Orthesis according to either claim 2 or claim 3, **characterised in that** the cam ring (38) has one or more stops (38c) for the rotor (42).

5. Orthesis according to any one of the preceding claims, **characterised in that** the joint rail (12) has a bearing member (34) which is arranged on the joint axle (18a) and on which the correction pad (22) is supported in an axially displaceable manner.

6. Orthesis according to claim 5, **characterised in that** the correction pad (22) has one or more, preferably cylindrical, bearing journal(s) (30) which is/are slidingly supported in holes (32) of the bearing member (34).

7. Orthesis according to claim 6, **characterised in that** the bearing journals (30) are provided at one end with a retention portion (36), on which the cam ring (38) is releasably fixed.

8. Orthesis according to any one of the preceding claims, **characterised in that** the rotor (42) has a ball-bearing roller.

9. Orthesis according to any one of the preceding claims, **characterised in that** the correction pad (22) has a rotary plate (26) having a padded coating (28).

10. Orthesis according to any one of the preceding claims, **characterised by** another joint rail (12) having a first and a second rail portion (14, 16).

## Revendications

1. Orthèse (10) destinée à stabiliser, guider et/ou soulager une articulation, comprenant
une attelle articulée (12) dotée d'une première et d'une deuxième partie d'attelle (14, 16), qui peuvent pivoter l'une par rapport à l'autre autour d'un axe d'articulation (18a), et une pelote de correction (22) qui, par un pivotement relatif des deux parties d'attelle (14, 16), peut être déplacée le long de l'axe d'articulation (18a) en direction de l'articulation à stabiliser pour s'appliquer contre celle-ci,
**caractérisée en ce qu'**une des deux parties d'attelle (14, 16) présente un élément courbe (38a) à came,
sachant que l'autre partie d'attelle respective (14, 16) présente une molette (42) guidée sur l'élément courbe à came,
et sachant que l'élément courbe (38a) à came ou la molette (42) est axialement bloqué(e) en position sur la pelote de correction (22).

2. Orthèse selon la revendication 1, **caractérisée en ce que** l'élément courbe (38a) à came est formé par une bague à came (38), qui est fixée de manière amovible sur la pelote de correction.

3. Orthèse selon la revendication 2, **caractérisée en ce que** la bague à came (38) peut, quant à sa position axiale et à sa position de rotation par rapport à la pelote de correction (22), être disposée d'une manière variable sur la pelote de correction (22).

4. Orthèse selon l'une des revendications 2 ou 3, **caractérisée en ce que** la bague à came (38) présente une ou plusieurs butées (38c) pour la molette (42).

5. Orthèse selon l'une des revendications précédentes, **caractérisée en ce que** l'attelle articulée (12) présente un élément (34) formant palier, disposé sur l'axe d'articulation (18a) et sur lequel la pelote de correction (22) est montée à translation axiale.

6. Orthèse selon la revendication 5, **caractérisée en ce que** la pelote de correction (22) présente un ou plusieurs tourillons (30) de préférence cylindriques, qui sont montés en contact glissant dans des perçages (32) de l'élément (34) formant palier.

7. Orthèse selon la revendication 6, **caractérisée en ce que** les tourillons (30) sont pourvus à une extrémité d'une partie de maintien (36), sur laquelle la bague à came (38) est fixée de manière amovible.

8. Orthèse selon l'une des revendications précédentes, **caractérisée en ce que** la molette (42) présente une roulette montée sur billes.

9. Orthèse selon l'une des revendications précédentes, **caractérisée en ce que** la pelote de correction (22) présente un plateau tournant (26) avec un revêtement de rembourrage (28).

10. Orthèse selon l'une des revendications précédentes, **caractérisée par** une attelle articulée supplémentaire (12) dotée d'une première et d'une deuxième partie d'attelle (14, 16).
